Europäisches Patentamt

European Patent Office (11) Publication number: **0 258 777**

Office européen des brevets **A1**

(12) **EUROPEAN PATENT APPLICATION**

(21) Application number: 87112256.0

(51) Int. Cl.4: **A61M 5/14 , A61M 25/00**

(22) Date of filing: 24.08.87

(30) Priority: 25.08.86 NL 8602154

(43) Date of publication of application:
09.03.88 Bulletin 88/10

(84) Designated Contracting States:
DE FR GB IT NL

(71) Applicant: CORDIS EUROPA N.V.
Oosteinde 8
NL-9300 AA Roden(NL)

(72) Inventor: Kroon, Robert c/o Cordis Europa,
N.V.
Oosteinde 8
NL-9300 AA Roden(NL)

(74) Representative: Kuhnen, Wacker & Partner
Schneggstrasse 3-5 Postfach 1729
D-8050 Freising(DE)

(54) Implantable dispenser for a liquid substance.

(57) An implantable dispenser (10) for a liquid substance, especially a liquid medicament, which dispenser comprises a housing (11) having a reservoir for the liquid substance, and an inlet port (23) sealed in liquid-tight fashion by a pad but accessible to a hypodermic needle, through which port the liquid substance can be applied from outside the body by means of the needle to the reservoir or can so be withdrawn therefrom. The inlet port (23) further includes a chamber (25) in hydraulic communication with the reservoir (27), which chamber is provided with a needle buffer plate (30) for limiting the penetration of the hypodermic needle into the inlet port, a normally closed valve means resting in sealing fashion on a valve seat and adapted to be opened, actuating means (34, 36) for maintaining the valve means in its normally closed position, and a liquid-impermeable, self-sealing pad (28) for sealing the chamber on the side remote from the reservoir, which pad is penetrable by the hypodermic needle.

FIG.3

## IMPLANTABLE DISPENSER FOR A LIQUID SUBSTANCE

The invention relates to a hand-operated, subcutaneously implantable dispenser for a liquid substance, especially a liquid medicament, which dispenser comprises a housing having a reservoir for the liquid substance, and an inlet port sealed in liquid-tight fashion by a pad but accessible to a hypodermic needle, through which port the liquid substance can be applied from outside the body by means of the needle to the reservoir or can so be withdrawn therefrom, which inlet port further includes a chamber in hydraulic communication with the reservoir, which chamber is provided with a needle buffer plate for limiting the penetration of the hypodermic needle into the inlet port, a normally closed valve means resting in sealing fashion on a valve set an adapted to be opened, actuating means for maintaining the valve means in its normally closed position, and a liquid-impermeable, self-sealing pad for sealing the chamber on the side remote from the reservoir, which pad is penetrable by the hypodermic needle.

U.S. Patent No. 4,557,722 discloses a subcutaneously implantable dispenser including an inlet port through which the medicament may be applied from outside the patient to a reservoir of the dispenser by means of a hollow needle or can so be withdrawn therefrom. The inlet port incorporates a number of functions to guarantee safety both when applying or withdrawing the medicament.

These functions primarily concern the liquid-tight sealing of the access to the interior of the dispenser in a general sense, and further the prevention of direct contact between the pressurized liquid medicament in the reservoir and the sealing pad by keeping the hydraulic connection between the chamber of the inlet port and the reservoir closed under pressure, as well as the limiting of the penetration of the hypodermic needle into the inlet port. The prior port comprises a special member for each of these functions. An elastically compressible sealing pad is used for the general sealing in liquid-tight fashion of the access to the interior of the dispenser, while a needle buffer plate of a rigid polymer material, such as a polysulfone material, is employed for limiting the penetration of the needle, and for keeping the hydraulic connection between the chamber in the inlet port and the reservoir normally closed there is provided a stop valve of a flexible material, such as a silicone rubber, in the chamber, which valve rests sealingly on a valve seat especially formed in the faces of the dispenser housing that define the sidewalls of the port chamber. The housing and hence the chamber sidewalls are of a bio-compatible, rigid material, for example a rigid polymer material such as a polysulfone material.

The needle buffer plate is functionally connected with the stop valve to the effect that when the buffer plate is moved, for example due to the needle being urged against it, the stop valve moves a corresponding distance with and in the same direction as the plate. To this end, the buffer plate includes a stem integrally formed thereon to anchor it to the stop valve. The assembly of the thus interconnected buffer plate and stop valve is constantly under pressure exerted, for example, by a helical spring, while the stop valve is retained on the valve seat by the spring pressure as well as the pressure exerted by the liquid medicament in the reservoir. In the prior inlet port the diameters of the needle buffer plate, the chamber, the stem and the stop valve are so dimensioned that, when depressing the buffer plate by means of a hollow needle inserted through the sealing pad, a passage to the reservoir is released as the liquid substance can flow past the edge of the buffer plate further into the chamber and via the space between the valve seat and the stop valve into the reservoir.

It is an object of the invention to provide a dispenser of the above type that includes an inlet port of simpler structure than the prior port as it employs a lesser number of co-acting components for performing the respective functions.

The dispenser is characterized in that the needle buffer plate coincides with the valve means and, as the valve means in its closed position, rests sealingly against the face of the sealing pad that faces the reservoir.

The invention is based on the concept that the use of the elastic properties of the sealing pad for the general sealing of the access to the interior of the dispenser need not interfere with the simultaneous use of these properties for other sealing purposes.

The needle buffer plate preferably has its face facing the sealing pad provided with a continuous upright flange preferably of a flexible material.

The invention will now be described in greater detail hereinafter with reference to a number of embodiments of the inlet port and in conjunction with the accompanying drawings, in which:

Fig. 1 shows in perspective top view a prior art dispenser for a liquid medicament which can be implanted subcutaneously in a patient for operaton by this patient;

Fig. 2 shown in enlarged cross-sectional view an embodiment of an inlet port fit for use in the dispenser shown in Fig. 1; and

Fig. 3 schematically shows in cross-sectional view a further embodiment of an inlet port fit for use in the dispenser of Fig. 1

Fig. 4 schematically shows in cross-sectional view a further embodiment of an inlet port fit for use in the dispenser of Fig. 1.

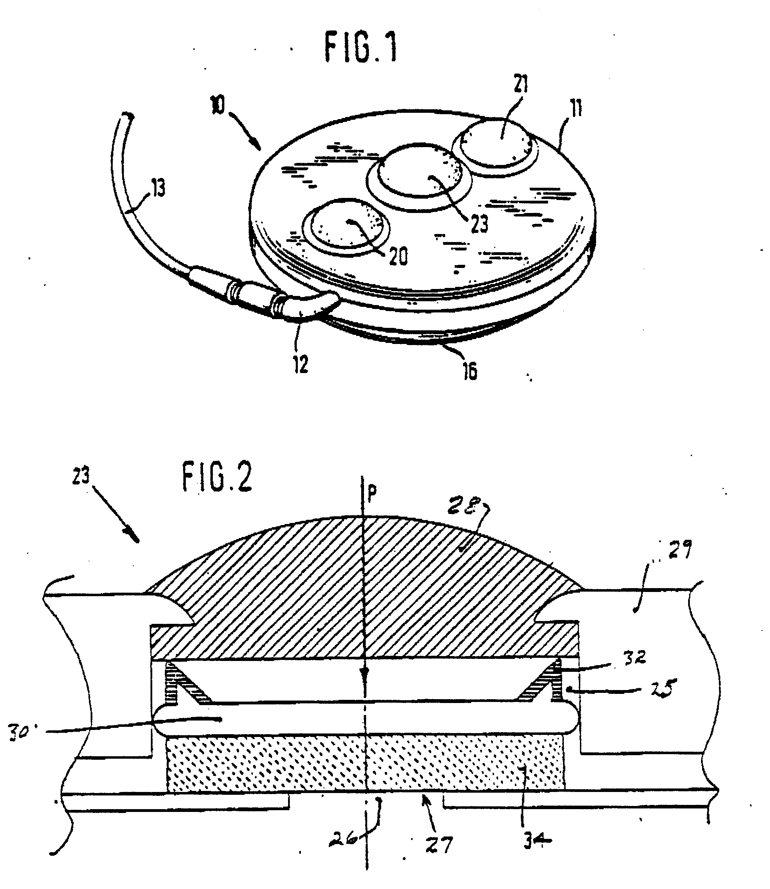

Fig. 1 illustrates a subcutaneously implantable, essentially disc-shaped dispenser 10 for a liquid medicament, comprising a housing 11 including a reservoir for the medicament. The reservoir has a flexible wall 16 constructed of Dacron reinforced silicone rubber. The liquid medicament contained in the reservoir is kept under pressure by means of flexible wall 16. Dispenser 10 further comprises a combination of co-acting pumps 20 and 21 operably from the outside through the skin of the patient by exerting pressure thereon to so apply doses of the medicament from the reservoir through the outlet port 12 and the flexible catheter 13 to the point in the patient's body where the medicament is to be administered.

An inlet port 23 through which the liquid medicament can be applied from outside the patient to the reservoir or, vice versa, can be withdrawn therefrom. Inlet port 23, in an embodiment as shown in Fig.2 includes a chamber 25 communicating through the channel 26 with the reservoir 27. Chamber 25 has its end remote from the reservoir sealed by a sealing pad 28 retained compressively in a recess formed in housing 29. Housing 29 is made of rigid polysulfone material. Sealing pad 28 is made of a silicone rubber and is penetrable by a hypodermic needle, the through-hole automatically closing upon retraction of the needle.

A needle buffer plate 30 of polysulfone material is shown having a sealing flange 32, in mounted on an open-cell sponge 34 likewise of a synthetic material, which is entrapped compressively within chamber 25. On account of the expansion force exerted sponge 34 on needle buffer plate 30, this plate is urged with its sealing flange 32 in liquid-tight fashion against pad 28. Upon insertion of the hollow needle of a syringe into and through sealing pad in a direction as indicated, for example, by arrow P, the needle will contact buffer plate 30 and depress it against the compressive force exerted by sponge 34. As a result, a passage is released between pad 28 and flange 32 and, when the syringe is emptied in the usual manner, the liquid medicament will flow past the edge of plate 30 and reach the reservoir via the open-cell sponge and channel 26. Upon retraction of the needle out of seating pad 28, sponge 34 will expand and urge buffer plate 30 with its sealing flange 32 against the bottom face of sealing pad 28 in liquid-tight fashion. Apart from an improved sealing effect when urged against pad 28, sealing flange 32 affords the advantage of additional safety as, when inserting the needle obliquely into and through pad 28, this flange reduces considerably the chances of the needle being pushed past the buffer plate. The use of the open-cell sponge 34 as the actuating means for urging needle buffer plate 30 in its normally closed position against pad 28 has an important, additional advantage as it acts as a filter for the liquid medicament.

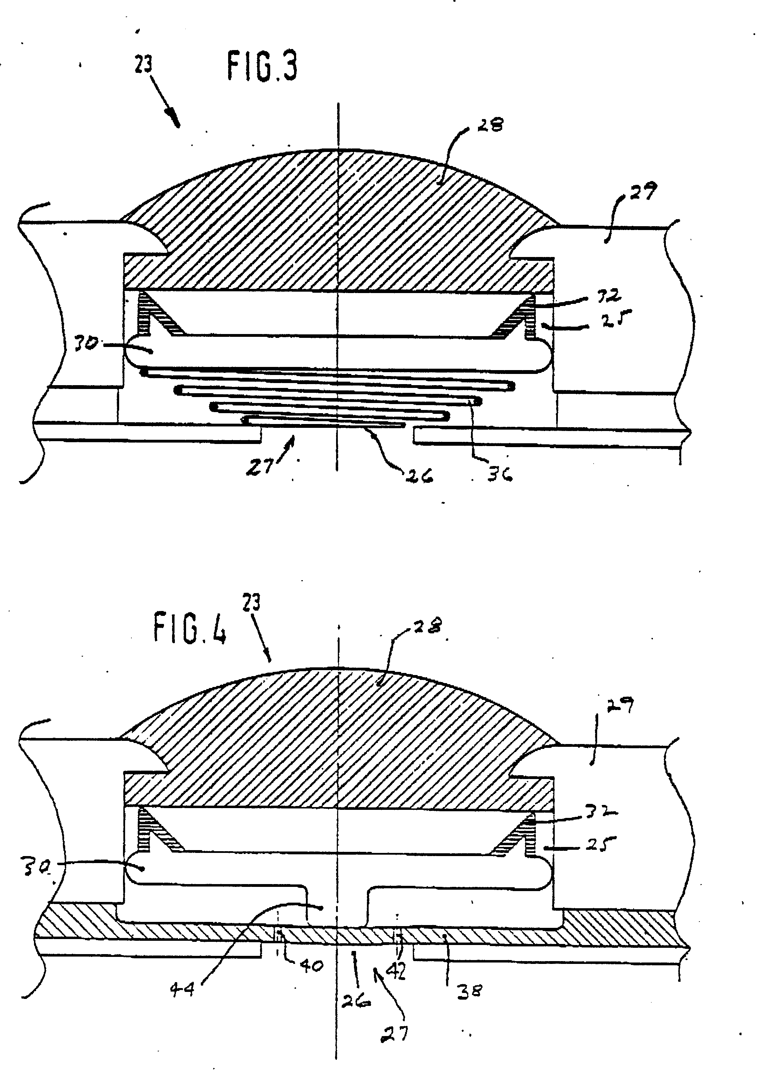

The embodiment of the inlet port shown in Fig. 3 is similar to that of Fig. 2 except for the fact that the sponge has been replaced by a helical spring 36. The spring 36 is another type of actuating means operative, just as sponge 34, to hold the needle buffer plate in its normally closed, sealing position against pad 28.

In the embodiment of the inlet port of the dispenser shown in Fig. 4, an elastic sheet 38 of silicone rubber fixedly clamped in the dispenser housing is used as the actuating means for keeping buffer plate 30 in its normal sealing position against pad 28. At channel 26 the elastic sheet 38 is provided with two through-channels 40, 42 through which the liquid medicament can enter into reservoir 27. When inserting the hollow needle into and through pad 28 to depress needle buffer plate 30, a projection 44 formed on plate 30 elastic sheet 38 stretches into the direction of reservoir 27 and a passage between pad 28 and sealing flange 32 is released. Upon retraction of the needle, the elasticity of sheet 38 ensures that the needle buffer plate is returned into its normally closed, sealing position.

As, in accordance with the invention, the functions of the needle buffer plate and the valve of the prior inlet port and hence these components proper coincide, there is no need for a structure involving a combination of a separate valve and a specially formed seat therefor, so that the structure of the inlet port according to the invention is considerably simpler than that of the prior port without the safety of the dispenser being impaired.

Self-evidently, modifications to the inlet port of the dispenser according to the invention as described above and shown in the accompanying drawings are possible without departing from the scope of the invention.

## Claims

1. A hand-operated, subcutaneously implantable dispenser for a liquid substance, especially a liquid medicament, which dispenser comprises a housing having a reservoir for the liquid substance, means for passing the liquid substance from the reservoir to the point in the body where the substance is to be administered, and an inlet port sealed in liquid-tight fashion by a pad but accessible to a hypodermic needle, through which port the liquid substance can be applied from outside the body by means of the needly to the reservoir or can so be withdrawn therefrom, which inlet port further includes a chamber in hydraulic communication with the reservoir, which chamber is provided with a needle buffer plate for limiting the penetration of the hypodermic needle into the inlet port, a normally closed valve means resting in sealing fashion on a valve seat and adapted to be opened, actuating means for maintaining the valve means in its normally closed position, and a liquid-impermeable, self-sealing pad . for sealing the chamber on the side remote from the reservoir, which pad is penetrable by the hypodermic needle, characterized in that the needle buffer plate coincides with the valve means and, as the valve means in its closed position, rests sealingly against the face of the sealing pad that faces the reservoir.

2. A dispenser according to claim 1, characterized in that the needle buffer plate has its face facing the sealing pad provided with a continuous upright flange.

3. A dispenser according to claim 2, characterized in that the upright flange is of flexible material.

4. A dispenser according to claim 1, characterized in that the sealing of the valve means against the sealing pad is achieved on account of the pressure exerted by a compressed, open-cell sponge.

# FIG.1

# FIG.2

FIG.3

FIG.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 502 123  (P. LORD)<br>* Page 8, line 25 - page 10, line 16; figure 2 * | 1,2 | A 61 M   5/14<br>A 61 M   25/00 |
| D,A | US-A-4 557 722  (HARRIS)<br>* Abstract; figures 4,5 * | 1 | |

**TECHNICAL FIELDS SEARCHED (Int. Cl.4)**

A 61 M

The present search report has been drawn up for all claims

| Place of search | Date of completion of the search | Examiner |
|---|---|---|
| THE HAGUE | 25-11-1987 | VANRUNXT J.M.A. |